# EUROPEAN PATENT SPECIFICATION

(11) **EP 0 394 278 B1**
(45) Date of publication and mention of the grant of the patent: **11.05.1994**
(21) Application number: 88907539.6
(22) Date of filing: 08.08.1988
(51) Int. Cl.: G03B 27/62

(54) **APPARATUS FOR PRINTING CUSTOMIZED PHOTOGRAPHIC PRINTS**
VORRICHTUNG ZUM KOPIEREN VON KUNDENANGEPASSTEN PHOTOGRAPHISCHEN KOPIEN
APPAREIL DE TIRAGE DE COPIES PHOTOGRAPHIQUES PERSONNALISEES

(30) Priority: 14.08.1987 US 85670
(43) Date of publication of application: 31.10.1990
(73) Proprietor: EASTMAN KODAK COMPANY, Rochester, New York 14650-2201 (US)
(72) Inventor: POHLMAN, James, Michael, Norwich CT 06360-9406 (US); MANICO, Joseph, Anthony, Rochester, NY 14618 (US)
(74) Representative: Lewandowsky, Klaus, Dipl.-Ing.
(86) International application number: US8802677
(87) International publication number: WO8901649

(56) References cited:
- DE-A- 3 232 254
- US-A- 2 153 212
- US-A- 3 694 079

## Description

### TECHNICAL FIELD

The present invention relates generally to a photographic printer for exposing a film image and a print image onto a photosensitive paper and more specifically to the making of customised photographic prints such as greeting cards.

### DISCLOSURE OF THE INVENTION

There exists a well recognized market for manufacturing customized greeting cards including personalized picture images provided by customers. Such greeting cards provide, for example, the opportunity for a customer to include a picture of his family on a holiday greeting card.

One method of producing such customized greeting cards comprises providing a pre-printed card including a cut-out or aperture into which a customer-supplied print can be mounted. A customer then purchases such a card, and inserts his own print into the cut-out. The resulting customized greeting card, however, does not provide a professional appearance. Further, such a greeting card is expensive, requiring a customer to purchase the pre-printed card and to supply a finished photographic print.

Another method of providing such customized greeting cards is shown in U.S. Patent No. 3,136,232 to Shoberg et al. The Schoberg et al. reference shows an easel including a contact printer situated on the edge thereof. The easel is used in combination with a photographic printer/enlarger, and functions to support a portion of photographic paper onto which a print is projected. The contact printer is used ot print customized artwork, such as a holiday greeting, onto the edge of the photographic paper portion. The result is a customized photographic print including a customer supplied picture image and a selected, customizing artwork.

Schoberg et al. suffers from the disadvantage of requiring the additional contact printer to print the customizing artwork. Such a contact printer is expensive and complex, requiring at a minimum a light source, power, a control mechanism for the light source, and accompanying hardware. The easel taught by Schoberg et al. thus adds significantly to the cost and maintenance of a basic photographic printer.

In US-A-3,694,079 an apparatus is described for printing identification card film negatives having a portrait area of low density and a data area of high density. When producing a print from such a negative by a single exposure, the exposure of the lower density area is variably reduced with respect to an adjacent or surrounding high density area through the use of polarizer filters.

It is the object of the present invention to provide a photographic printer of the generic type such that one of a plurality of print images can be combined with a film image and the light passing through each print image can be filtered separately.

In accordance with the invention, this object is essentially attained by both rotatably mounted print image supporting means and filtering means.

New and improved apparatus is provided for producing customized photographic prints, each customized print including a customer-supplied picture image and a selectable, customizing artwork such as a holiday greeting. The photographic printer includes means for projecting light through a film image to expose said film image onto a photographic paper. In accordance with the present apparatus, a printable, selectable artwork is supported in the path of the projected light so as to expose the artwork onto the photographic paper in a selected position relative to the exposure of the film image.

In a preferred embodiment of the invention means are provided for supporting a plurality of printable artworks and for permitting an operator to position a selected one of the artworks substantially coplanar with and adjacent to the customer-supplied film image. Further provided are means for supporting a plurality of neutral density filters and for permitting an operator to position a selected one of the neutral density filters in registry with the selected artwork.

### BRIEF DESCRIPTION OF THE DRAWINGS

While the specification concluded with claims defining the features of the invention that are regarded as novel, it is believed that the invention, together with further objects thereof, will be better understood from a consideration of the following description in conjunction with the drawing figures, in which like reference numerals are carried forward, and in which:
Fig. 1 shows a schematic view of a photographic printer constructed in accordance with the present invention;
Figs. 2, 3, and 4, show top, side section and bottom views, respectively, of the custom film gate of Fig. 1; and
Figs. 5 and 6 show drawings representative of custom prints produced in accordance with the present invention.

### MODES OF CARRYING OUT THE INVENTION

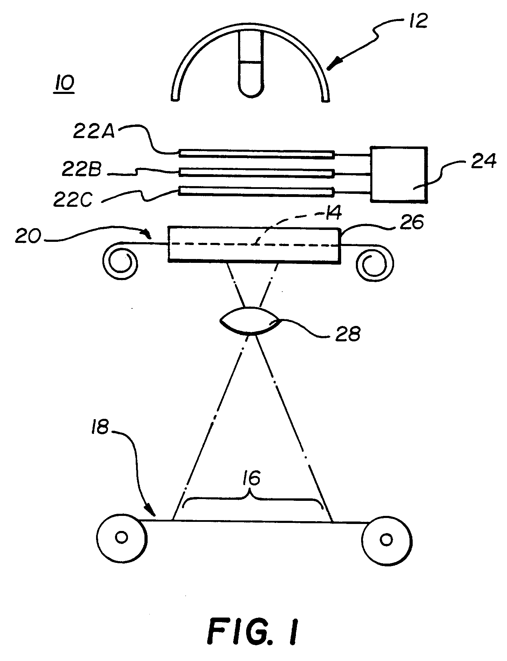

Referring now to the drawings, Fig. 1 shows a printer 10 including a light source 12 for projecting light through a selected film image or negative 14 to form an exposure 16 on a portion of a roll of photographic paper 18. Negative 14 comprises a selected one of a disc or roll 20 of customer-supplied negatives.

In accordance with standard features known to those skilled in the art, printer 10 further includes additive or subtractive color filters 22A, 22B, 22C, connected to a controller 24, for controlling the exposure times of the various colors in negative 14. A new and improved film gate 26, described in detail below, is provided for supporting negative 14 and a selectable, printable artwork (not visible in Fig. 1) in the path of the light projected by light source 12. A printing lens 28 is disposed intermediate film gate 26 and photographic paper 18 for imaging film negative 14 and the artwork into a plane of focus on the photographic paper.

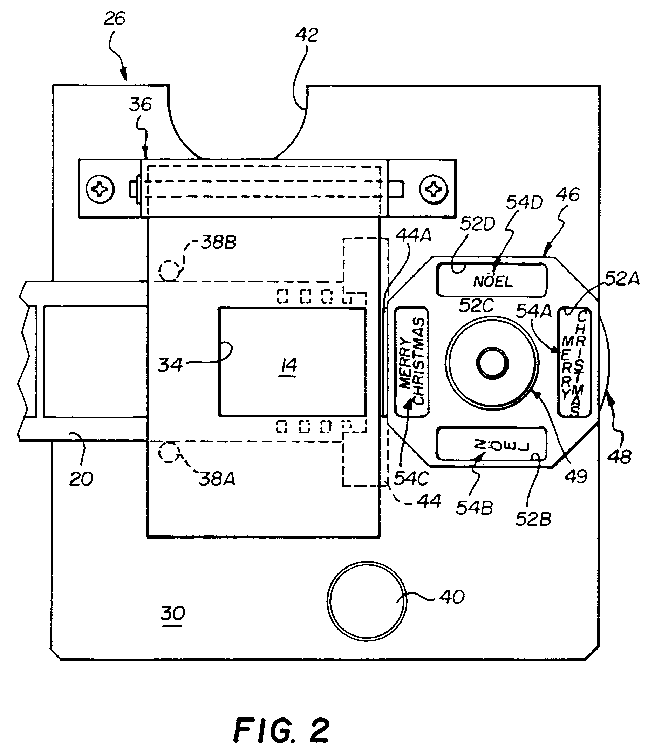

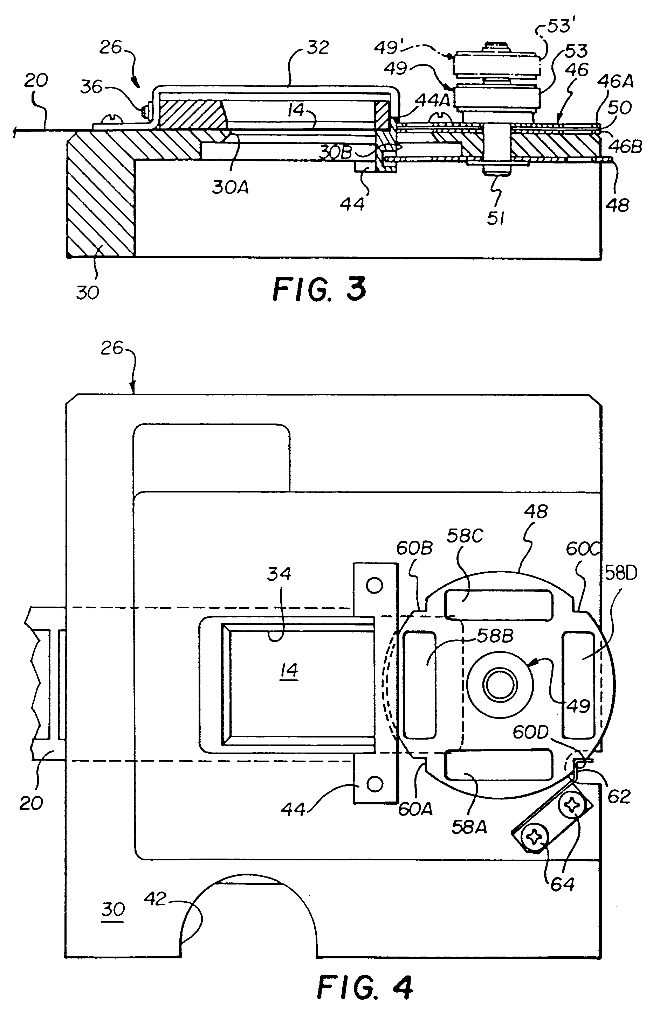

Referring now to Figs. 2, 3, and 4, film gate 26 comprises an opaque base 30 defining first and second rectangular apertures 30A, 30B (Fig. 3). In the embodiment shown and described herein, aperture 30A is approximately the size of an exposed 35mm negative. Aperture 30B is approximately one fourth the size of aperture 30A, the two apertures being disposed generally adjacent one-another. It will be understood that the size of apertures 30A, 30B is determined by the size of negative 14 and the customized artwork, respectively, the artwork being shown and described in detail below.

Supported on an upper side of base 30 is a frame 32 defining a printing aperture 34. Frame 32 is mounted to base 30 via a hinge 36 such that, with frame 32 in the closed position (as shown in Figs. 2-4), printing aperture 34 is substantially in registry with base aperture 30A. An opposing pair of guide pins 38A, 38B are mounted on base 30 underneath frame 32 and spaced the width of filmstrip 20. As described in detail below, guide pins 38A, 38B are used to position negative 14 in alignment with apertures 30A, 34. A mounting aperture 40 and notch 42 are provided for securing film gate 26 in position with mating features (not shown) on printer 10. A bracket 44 is mounted on the underside of base 30 and includes a bar-shaped stop 44A projecting through the base and adjoining a side of frame 32.

Further provided with film gate 26 are generally opposing, rotatable message and filter wheels, 46, 48, respectively. A knob assembly 49 includes a shaft 51 extending through message wheel 46 and filter wheel 48 and being secured to base 30, with the message and filter wheels being rotatable about the axis of the shaft. A thumbwheel 53 is secured to shaft 51 by a threaded screw connection (not visible). As shown in solid line in Fig. 3, with thumbwheel 53 screwed tightly onto shaft 51, message and filter wheels 46, 48, respectively, are positioned adjoining opposite sides of base 30, with a selected side of the message wheel abutting stop 44A.

As is best shown in Fig. 3, message wheel 46 overlies the top of base 30 adjacent stop bar 44A, and comprises a pair of opposing, octagonally-shaped plate, 46A, 46B, sandwiching an intermediate, changeable, artwork medium 50. Plates 46A, 46B each define four apertures, the apertures being in registry to provide four apertures 52A, 52B, 52C, 52D extending through message wheel 46 and positioned adjacent respective octagonal sides thereof. Artwork medium 50 comprises, for example, a film base including four separate messages 54A, 54B, 54C, 54D, each message being centered in a corresponding aperture 52A-52D. Plates 46A, 46B comprise an opaque, relatively stiff material for supporting artwork medium 50, for example a metal such as aluminum. Message wheel 46 is sized such that, in the operating position shown in solid line in Figs. 2-4, a selected edge adjacent a selected message 54A-54D can be positioned snugly adjoining stop bar 44A. The selected message 54A-54D is concomitantly positioned in registry with aperture 30B.

Filter wheel 48, as is best shown in Fig. 4, comprises a circular portion of film base having four neutral density filter regions 58A, 58B, 58C, 58D disposed thereon. As will be discussed in further detail below, each of filter regions 58A-58D comprises a different neutral density characteristic. Filter wheel 48 includes four notches 60A, 60B, 60C, 60D spaced about the periphery thereof. A detent 62 is secured to base 30 with screws 64 for engaging a selected notch 60A-60D. Filter wheel 48 is sized, and neutral density filter regions 58A-58D are spaced, such that a selected neutral density filter region can be positioned in registry with aperture 30B and hence in registry with a selected message 54A-54D.

In operation, filmstrip 20 is trimmed such that negative 14 is on an end thereof. Frame 32 is pivoted away from base 30, and film strip 20 is positioned between pins 38A, 38B and abutting stop 44A. Frame 32 is then pivoted down to hold negative 14 in place. Negative 14 is thus in registry with apertures 30A and 34 as shown in Figs. 2-4.

An operator then selects a desired message 54A-54D to be printed with negative 14, and rotates message wheel 46 until that message is positioned adjoining stop 44A. As illustrated in dot-dashed line, and indicated by primed reference numerals in Fig. 3, message wheel 46 is rotated by unscrewing thumbwheel 53 to the 53' position on shaft 51, lifting the message wheel clear of stop 44A, and rotating the message wheel until the selected message 54A-54D is positioned adjacent the stop. Message wheel 46 is then lowered back onto base 30, and the thumbwheel tightened back down on shaft 51 to hold the message wheel securely in position (as shown in solid line in Figs. 2-4). The selected message, for example message 54C as shown in Fig. 2, is thus situated in registry with aperture 30B in base 30.

In addition to selecting a message 54A-54D, the operator further selects a neutral density filter region 58A-58D to position in registry with the selected message. The selected neutral density filter is positioned by rotating filter wheel 48, with detent 62 engaging the appropriate notch 60A-60D to hold the selected neutral density filter securely in position. It will be appreciated that the function of neutral density filter regions 58A-58D are to provide proper exposure of the selected artwork 54A-54D. The selection of a proper neutral density filter will, of course, be a function of the characteristics of both negative 14 and the selected artwork 54A-54D, and is well within the purview of those skilled in the art.

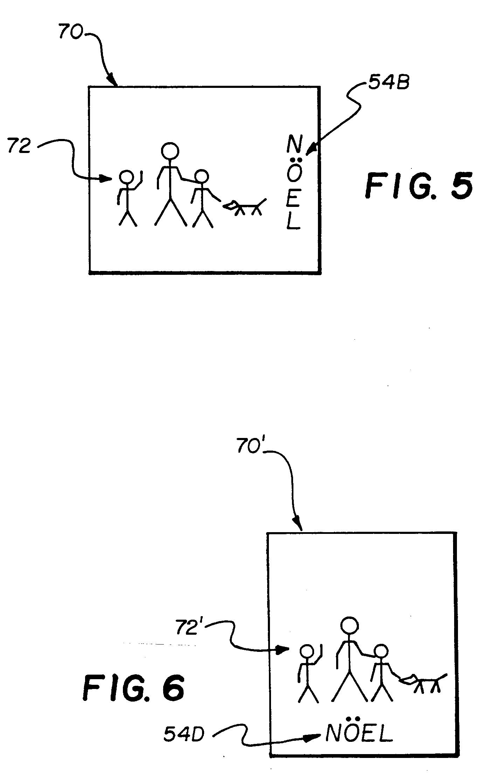

After positioning negative 14, a selected artwork 54A-54D, and a selected neutral density filter region 58A-58D as described above, an exposure is made in printer 10 (Fig. 1) in accordance with standard printing procedures. Referring now to Figs. 5 and 6, the final, customized print 70, 70' will comprise the print of the customer supplied negative 14, indicated generally at 72, 72', adjoined by the operator-selected artwork (message 54B in Fig. 5 and message 54D in Fig. 6).

While message wheel 46 has been described herein as comprising relatively simple, stencil-type messages 54A-54D, it will be understood that the invention is not so limited. Messages 54A-54D could comprise, for example, more complex artworks such as cartoon-type figures or even photographic scenes. It will be appreciated that the number of artworks 54 and neutral density filters 58 are not limited to the four shown. Further, it will be understood that it will be necessary to select lens 28 to have a focal length appropriate to image both negative 14 and the selected artwork 54 onto photographic paper 18. Such lenses are readily commercially available, and the selection of such a lens is well within the purview of those skilled in the art.

There is thus provided a color photographic printer, including a customized print gate, for producing customized prints including selectable artworks in combination with a customer-supplied image. The invention may be implemented in substantially any printer by incorporating the customized film gate. Further, the invention provides the substantial advantage of utilizing all of the major components already present in a printer, including the light source, exposure control apparatus, printing lens, and paper transport and handling mechanism.

While a preferred embodiment of the invention has been illustrated and described, it will be clear that the invention is not so limited. Numerous modification, changes, variations, substitutions and equivalents will occur to those skilled in the art without departing from the scope of the present invention as claimed.

## Claims

1. A photographic printer for exposing a film image (14) and a print image (54A, 54B, 54C, 54D) onto a photosensitive paper (18), said device comprising
base means (26, 30) for supporting the film image (14) having an opening (30B, 34) permitting the simultaneous projection of said film image (14) and print image (54A, 54B, 54C, 54D);
means (46, 46A, 46B) for supporting the print image (54A, 54B, 54C, 54D);
a single light source (12) for projecting light through the film image (14) and through the print image (54A, 54B, 54C, 54D) to project the images (14, 54A, 54B, 54C, 54D) onto the photosensitive paper (18);
means (48; 58A, 58B, 58C, 58D) for filtering the light prior to reaching the photosensitive paper (18), and
means (51, 53) for connecting the filtering means (48; 58A, 58B, 58C, 58D) to the base means (26, 30) in a manner that it is movable to a location in general alignment with an image area (52A, 52B, 52C, 52D) in order to filter the light travelling through said area onto the photosensitive paper (18),
**characterized in that** the print image supporting means (46, 46A, 46B) supports a plurality of print images (54A, 54B, 54C, 54D) and in that
both the print image supporting means (46, 46A, 46B) and the filtering means (48, 58A, 58B, 58C, 58D) are rotatably connected to the base means (26, 30) and are rotatable to position one of the plurality of print images to a location in general alignment with the opening (30B) adjacent to the film image (14) to allow the light to expose the print image (54A, 54B, 54C, 54D) onto the photosensitive paper (18).

2. A photographic printer according to claim 1, **characterized in that** the base means (26) includes a first opening (34) over which the film image (14) is supported, and a second opening (30B) over which the print image (54A, 54B, 54C, 54D) is supported.

3. A photographic printer according to claim 1 or 2, **characterized by** second means (22A, 22B, 22C) for filtering the light which exposes the film image (14) onto the photosensitive paper (18).

4. A photographic printer according to claim 3, **characterized by**
shaft means (51), having an axis which extends through the base means (26);
means for connecting the print image supporting means (46) to the shaft means (51) in a manner that the print image supporting means (46) is rotatable about the shaft axis to a first location, and
means for connecting the filtering means (48) in a manner that the filtering means (48) is rotatable about the shaft axis to a second location.

5. A photographic printer according to claim 4, **characterized in that** the print image supporting means (46 46A, 46B) is located at a first side of the base means (26) and the filtering means (48, 58A, 58B, 58C, 58D) is located at an opposite second side of the base means (26).

6. A photographic printer according to claim 5, **characterized in that** the print image supporting means (46) is located coplanar with a plane of the film image (14).

7. A photographic printer according to claim 6, **characterized in that** the base means (26) includes a first alignment member (44A) and the print image supporting means (46) includes a second alignment member.

8. A photographic printer according to claim 7, **characterized in that** the connecting means includes
means for allowing movement of the print image supporting means (46) away from the base means (26) so that the print image supporting means (46) can be moved about the shaft axis to align the first and second alignment member as well as
means for allowing movement of the print image support means (46) towards the base means (26) when the first alignment member (44A) and the second alignment member are in alignment so that the first alignment member (44A) and the second alignment member can be engaged in order to secure the print image (54A, 54B, 54C, 54D) in general alignment with the second opening (30B).

9. A photographic printer according to claim 8, **characterized in that** the first alignment member (44A) extends upwardly from the base means (26) to engage the second alignment member in the first position and the connecting means includes means (49) for moving the print image supporting means (46) away from the base means (26) or the shaft means (51) so that the second alignment member is clear of the first alignment member (44A) to permit movement of the print image support means (46) about the shaft axis.

## Patentansprüche

1. Fotografischer Printer zum Belichten eines Filmbildes (14) und eines Textbildes (54A, 54B, 54C, 54D) auf ein lichtempfindliches Papier (18), mit
einer Auflage (26, 30), auf der das Filmbild (14) aufliegt und die eine Öffnung (30B, 34) aufweist, die die gleichzeitige Projektion des Filmbildes (14) und des Textbildes (54A, 54B, 54C, 54D) erlaubt,
Mitteln (46, 46A, 46B) für die Auflage des Textbildes (54A, 54B, 54C, 54D),
einer einzigen Lichtquelle (12), die Licht durch das Filmbild (14) und das Textbild (54A, 54B, 54C, 54D) und damit die Bilder (14, 54A, 54B, 54C, 54D) auf das fotografische Papier (18) projiziert,
Mitteln (48; 58A, 58B, 58C, 58D), die das Licht filtern, ehe es auf das fotografische Papier (18) auftrifft, und
Mitteln (51, 53) die die das Licht filternden Mittel (48; 58A, 58B, 58C, 58D) derart mit der Auflage (26, 30) verbinden, daß diese an einen im allgemeinen mit einem Bildbereich (52A, 52B, 52C, 52D) ausgerichteteten Ort bewegbar sind, so daß das durch diesen Bereich auf das lichtempfindliche Papier (18) fallende Licht filterbar ist,
**dadurch gekennzeichnet, daß**
auf den Mitteln (46, 46A, 46B) für die Auflage des Textbildes eine Vielzahl von Textbildern (54A, 54B, 54C, 54D) aufliegt und daß diese Mittel (46, 46A, 46B) und die Mittel (48; 58A, 58B, 58C, 58D) zum Filtern des Lichts drehbar mit der Auflage (26, 30) derart verbunden sind, daß eines aus der Vielzahl von Textbildern an einem im allgemeinen mit der Öffnung (30B) ausgerichteten, dem Filmbild (14) benachbarten Ort so positioniert ist, daß das Licht das Textbild (54A, 54B, 54C, 54D) auf das lichtempfindliche Papier (18) belichtet.

2. Fotografischer Printer nach Anspruch 1, dadurch gekennzeichnet, daß die Auflage (26) eine erste Öffnung (34) aufweist, über der das Filmbild (14) liegt, sowie eine zweite Öffnung (30B), über der das Textbild (54A, 54B, 54C, 54D) liegt.

3. Fotografischer Printer nach Anspruch 1 oder 2, gekennzeichnet durch zweite Mittel (22A, 22B, 22C), die das das Filmbild (14) auf das lichtempfindliche Papier (18) belichtende Licht filtern.

4. Fotografischer Printer nach Anspruch 3, gekennzeichnet durch
eine Welle (51) mit einer sich durch die Auflage (26) erstreckenden Achse,
Mittel, die die Mittel (46) für die Auflage des Textbildes derart mit der Welle (51) verbinden, daß die Mittel (46) um die Welle in eine erste Position drehbar sind, und
Mittel, die die Mittel (48) zum Filtern des Lichts derart miteinander verbinden, daß die Mittel (48) um die Welle in eine zweite Position drehbar sind.

5. Fotografischer Printer nach Anspruch 4, dadurch gekennzeichnet, daß die Mittel (46, 46A, 46B) für die Auflage des Textbildes auf einer ersten Seite der Auflage (26) und die Mittel (48, 58A, 58B, 58C, 58D) zum Filtern des Lichts auf einer gegenüberliegenden zweiten Seite der Auflage (26) angeordnet sind.

6. Fotografischer Printer nach Anspruch 5, dadurch gekennzeichnet, daß die Mittel (46) für die Auflage des Textbildes in derselben Ebene angeordnet sind wie das Filmbild (14).

7. Fotografischer Printer nach Anspruch 6, dadurch gekennzeichnet, daß die Auflage (26) ein erstes Ausrichtelement (44A) und die Mittel (46) für die Auflage des Textbildes ein zweites Ausrichtelement aufweisen.

8. Fotografischer Printer nach Anspruch 7, dadurch gekennzeichnet, daß die Verbindungsmittel
eine Vorrichtung aufweisen, die eine Bewegung der Mittel (46) für die Auflage des Textbildes weg von der Auflage (26) erlaubt, so daß die Mittel (46) um die Welle herum bewegbar sind und dabei das erste und zweite Ausrichtelement ausrichten, und
eine Vorrichtung umfassen, die eine Bewegung der Mittel (46) für die Auflage des Textbildes zur Auflage (26) hin erlauben, wenn das erste (44A) und das zweite Ausrichtelement ausgerichtet sind, so daß das erste (44A) und das zweite Ausrichtelement in Eingriff bringbar sind und dadurch das Textbild (54A, 54B, 54C, 54D) in allgemeiner Ausrichtung mit der zweiten Öffnung (30B) gehalten ist.

9. Fotografischer Printer nach Anspruch 8, dadurch gekennzeichnet, daß sich das erste Ausrichtelement (44A) von der Auflage (26) aus nach oben erstreckt und an dem zweiten Ausrichtelement in der ersten Position angreift und daß die Verbindungsmittel eine Vorrichtung (49) aufweisen, die die Mittel (46) für die Auflage des Textbildes von der Auflage (26) oder von der Welle (51) weg bewegt, so daß das zweite Ausrichtelement sich nicht mehr in Eingriff mit dem ersten Ausrichtelement (44A) befindet und dadurch die Mittel (46) um die Welle bewegbar sind.

## Revendications

1. Dispositif d'impression photographique pour exposer une image de film (14) et une image imprimée (54A, 54B, 54C, 54D) sur un papier photosensible (18), le dispositif comprenant :
un support (26, 30) supportant l'image de film (14) et ayant une ouverture (30B, 34) qui permet de projeter simultanément l'image de film (14) et l'image imprimée (54A, 54B, 54C, 54D) ;
un moyen (46, 46A, 46B) pour supporter l'image imprimée (54A, 54B, 54C, 54D) ;
une source de lumière unique (12) pour projeter la lumière au travers de l'image de film (14) et de l'image imprimée (54A, 54B, 54C, 54D) afin de former les images (14, 54A, 54B, 54C, 54D) sur le papier photosensible (18),
un moyen (48, 58A, 58B, 58C, 58D) de filtrage de la lumière avant qu'elle n'atteigne le papier photosensible (18), et
un moyen (51, 53) de connection du moyen de filtrage (48, 58A, 58B, 58C, 58D) au support (26, 30) de sorte que le moyen de connection puisse être déplacé pour être aligné avec une zone image (52A, 52B, 52C, 52D) afin de filtrer la lumière qui passe au travers de la zone pour exposer le papier photosensible (18),
caractérisé en ce que
le moyen pour supporter l'image imprimée (46, 46A, 46B) supporte une pluralité d'image imprimée (54A, 54B, 54C, 54D) et en ce que
le moyen pour supporter l'image imprimée (46, 46A, 46B) et le moyen de filtrage (48, 58A, 58B, 58C, 58D) sont connectés au support (26, 30) de façon à être mobile autour d'un axe et sont mis en rotation pour aligner une des images imprimées avec l'ouverture (30B) adjacente à l'image de film (14) pour permettre à la lumière d'exposer l'image imprimée (54A, 54B, 54C, 54D) sur le papier photosensible (18).

2. Dispositif d'impression photographique selon la revendication 1, dans lequel le support (26) comprend une première ouverture (34) sur laquelle on dispose l'image de film (14) et une seconde ouverture (30B) sur laquelle on dispose l'image imprimée (54A, 54B, 54C, 54D).

3. Dispositif d'impression photographique selon la revendication 1 ou 2, comprenant un second moyen (22A, 22B, 22C) de filtrage de la lumière qui expose l'image de film (14) sur le papier photosensible (18).

4. Dispositif d'impression photographique selon la revendication 3, comprenant
un arbre (51) ayant un axe qui passe à travers le support (26) ;
un moyen de connection du moyen de support de l'image imprimée (46) à l'arbre (51) de sorte que le moyen de support de l'image imprimée (46) soit mobile autour de l'axe de l'arbre dans une première position, et
un moyen de connection du moyen de filtrage (48) de sorte que le moyen de filtrage (48) soit mobile autour de l'axe de l'arbre dans une seconde position.

5. Dispositif d'impression photographique selon la revendication 4, dans lequel le moyen pour supporter l'image imprimée (46, 46A, 46B) est situé sur un premier coté du support (26) et le moyen de filtrage (48, 58A, 58B, 58C, 58D) est situé sur le second coté opposé du support (26).

6. Dispositif d'impression photographique selon la revendication 5, dans lequel le moyen pour supporter l'image imprimée (46) est situé de façon coplanaire par rapport à un plan de l'image de film (14).

7. Dispositif d'impression photographique selon la revendication 6, dans lequel le support (26) comprend un premier organe d'alignement (44A) et le moyen pour supporter l'image imprimée (46) comprend un second organe d'alignement.

8. Dispositif d'impression photographique selon la revendication 7, caractérisé en ce que le moyen de connection comprend
un moyen permettant l'éloignement du moyen pour supporter l'image imprimée (46) du support (26) de sorte que le moyen pour supporter l'image imprimée (46) puisse être mis en mouvement autour de l'axe de l'arbre pour aligner le premier et le second organe d'alignement, et
un moyen permettant le rapprochement du moyen pour supporter l'image imprimée (46) vers le support (26) lorsque le premier et le second organe d'alignement (44A) sont alignés de sorte que l'on puisse engager le premier et le second organe pour immobiliser l'image imprimée (54A, 54B, 54C, 54D) alignée avec la seconde ouverture (30B).

9. Dispositif d'impression photographique selon la revendication 8, dans lequel le premier organe d'alignement (44A) s'étend au-dessus du support (26) pour engager le second organe d'alignement dans la première position et le moyen de connection comprend un moyen (49) pour éloigner le moyen pour supporter l'image imprimée (46) du support (26) ou de l'axe (51) de sorte que le second organe d'alignement soit dégagé du premier organe d'alignement (44A) pour permettre le mouvement du moyen pour supporter l'image imprimée (46) autour de l'axe de l'arbre.
